# EUROPEAN PATENT APPLICATION

(11) **EP 2 377 514 A2**
(43) Date of publication of application: **19.10.2011**
(21) Application number: 11003295.0
(22) Date of filing: 19.04.2011
(51) Int. Cl.: A61K 9/00, A61K 31/135, A61K 31/167, A61K 47/02, A61K 47/10, A61K 47/18

(54) **Liquid parenteral formulation comprising a tramadol material and paracetamol**

(30) Priority: 19.04.2010 GR 2010100233
(71) Applicant: Uni-Pharma Kleon Tsetis Pharmaceutical Laboratories S.A., 145 64 Kifissia (GR)
(72) Inventor: Ioulia, Tseti, 145 64 Kifissia (GR)
(74) Representative: Wibbelmann, Jobst

(57) **Abstract**

The present invention provides a liquid parenterally deliverable formulation comprising a tramadol material, particularly tramadol HCl, and paracetamol and its use in treating conditions or disorder wherein an urgent treatment with analgesics is indicated.

This formulation is particularly useful whenever fast relief is required in lesser time, especially in acute or chronic painful conditions.

The formulation of the present invention is stable upon storage at room temperature and in refrigerated temperature.

## Description

### Field of the invention

The present invention relates to a liquid parenterally deliverable pharmaceutical formulation, particularly to a liquid parenterally deliverable formulation comprising a tramadol material and paracetamol, and more particularly to a liquid parenterally deliverable formulation, comprising tramadol as its pharmaceutically acceptable hydrochloride and paracetamol.

The invention also relates to a process of preparing a liquid parenterally deliverable formulation comprising a tramadol material and paracetamol, in particular to a process of preparing a liquid parenterally deliverable formulation comprising tramadol as its pharmaceutically acceptable hydrochloride and paracetamol.

In addition, there is also provided the therapeutic dosage form and the mode of administration of treating a subject having a condition or disorder, wherein treatment with a potent analgesic is indicated.

### Background of the invention

Paracetamol (acetaminophen) is a known painkiller and is a water insoluble, non-opiate, no salicylate compound, endowed with centrally acting analgesic effects.

A variety of paracetamol pharmaceutical formulations for oral, rectal and cutaneous administration are known. Pharmaceutical formulations comprising combination formulations of paracetamol with other active components like analgesics, antihistamines, antitussives, decongenstants etc. in the form of tablets, suspensions, elixirs and suppositories have also been provided.

Tramadol ((±)-(*1R**,*2R**)-2-(Dimethylaminomethyl) -1-(3-methoxyphenyl)-cyclohexanol) is a white, odorless, water soluble component. It is centrally acting synthetic opiate analgesic compound, related to morphine, but not as strong as a painkiller. It is available as its pharmaceutically acceptable hydrochloric salt, in one or more forms like tablets, capsules, oral solutions and suppositories.

The combination of paracetamol with tramadol HCl was not commonly available for prescription, but has recently become available in USA and Europe as an orally administered fixed combination in the form of film coated and effervescent tablets comprising 37,5 mg of tramadol HCl and 325 mg paracetamol.

Up to the present day, it is not known whether a formulation comprising tramadol HCl material and paracetamol has ever been authorized and/or marketed in the form of a liquid parenterally deliverable pharmaceutical formulation such as injectable solutions and/or injectable suspensions.

US Reissued Patent 39221 E discloses a formulation comprising tramadol HCl material with paracetamol, which when combined in certain rations exhibits synergistic analgesic effects.

It also disclosed that pharmaceutical formulations comprising tramadol HCl material and paracetamol can be prepared according to conventional pharmaceutical compounding techniques in a variety of forms depending on the form desired for administration e.g. intravenous, oral or parenteral.

Parenteral drug formulations have become a very important component in the arsenal of available drug delivery options, particularly for drugs having analgesic effect. Parenteral routes of administration, including subcutaneous, intramuscular and intravenous injection, offer numerous benefits over oral delivery in particular situations, for a wide variety of drugs. For example, parenteral administration of a drug typically results in attainment of a therapeutically effective blood serum concentration of the drug in a shorter time than is achievable by oral administration. This is especially true for intravenous injection, whereby the drug is placed directly in the bloodstream.

Parenteral administration can also result in more predictable blood serum concentrations of a drug, because losses in the gastrointestinal tract due to metabolism, binding to food and other causes are eliminated. For similar reasons, parenteral administration often permits dose reduction. Parenteral administration is generally the preferred method of drug delivery in emergency situations and is also useful in treating subjects who are uncooperative, unconscious, or otherwise unable or unwilling to accept oral medication and where a faster relief is required in lesser time, specially in acute and chronic painful condition.

If a parenteral drug formulation is to be prepared, it is preferable from patient convenience and safety standpoints that such a formulation be a ready-to-use formulation. Ready-to-use and dilutable liquid parenteral formulations can also be advantageous from a manufacturing standpoint by avoiding expensive lyophilization and/or other similar manufacturing steps.

Hence, in view of the above mentioned relevance to the mode of medicines administration, there is a perceived need for development of a tramadol material-paracetamol, in particular a tramadol HCl-paracetamol, combination formulation in stable injection form and a method of preparing the same suitable of treating a subject having a condition or disorder wherein a very urgent treatment with analgesics is indicated, this very urgent treatment being given by administering tramadol HCl-paracetamol combination formulation in an injectable form.

The present invention discloses a liquid parenterally deliverable pharmaceutical formulation and the preparation of a stable liquid parenterally deliverable pharmaceutical formulation comprising a tramadol material, in particular tramadol HCl, and paracetamol, in particular comprising the recently authorized therapeutical dosage form of 37,5 mg tramadol HCl and 325 mg paracetamol.

### Objects and advantages of the invention

Accordingly, the objects and advantages of the present invention are the following:
An object of the present invention is to provide a tramadol, in particular tramadol HCl, -paracetamol combination formulation in a liquid parenterally deliverable form.
Other object of the present invention is to provide a formulation, which is convenient to use and stable for long time.

Another object is the provide a tramadol, in particular tramadol HCl, -paracetamol injection so that an effective therapeutic concentration in serum is obtained in a short time.

Yet another object of the present invention is to provide a tramadol, in particular tramadol HCl, -paracetamol injection, which is safe, and has fast and potent effects.

A further object of the present invention is to develop a process for a liquid parenterally deliverable pharmaceutical formulation comprising a tramadol material, in particular tramadol HCl, and paracetamol avoiding expensive manufacturing steps.

### Description of the invention

The injectable formulation of the present invention fulfils the need for an alternate route of administration when a non-oral route is necessary or preferable.

According to a first aspect, there is provided a liquid parenterally deliverable formulation comprising a tramadol material and paracetamol. In a preferred embodiment, the tramadol material is a tramadol salt, in particular selected from tramadol hydrochlorid, tramadol hydroiodide, tramadol tartrate, tramadol citrate, tramadol methane sulphonate, and any combination thereof. Particularly, the tramadol material is tramadol hydrochlorid.

Typically, the formulation comprises from 20 mg to 50 mg, particularly 30 mg to 40 mg, and more particularly 37,5 mg tramadol material. Typically, the formulation comprises from 200 mg to 500 mg, particularly from 300 mg to 400 mg, and more particularly 325 mg paracetamol. According to the present invention it is therewith realizable to prepare a homogenous, clear and stable liquid pharmaceutical, which is parenterally deliverable and comprises the authorized fixed combination formulation of 325 mg paracetamol and 37,5mg tramadol HCl.

The main attempt in carrying into effect the liquid parenterally deliverable form of the tramadol material-paracetamol formulation was connected with paracetamol's poor water solubility. Therefore, the inventive study examined the solubilization possibilities of paracetamol, using a series of pure solvents and solvents mixtures. Water, alcohols, glycols glycerol, glycerol formal and polyethylene glycols, PEG 300 or PEG 400, were used as solvents and water-ethanol, glycerol-ethanol and polyethylene glycol-ethanol, were used as mixed solvent systems. Lower alcohols, higher glycols and PEG 400 were found to be good solvents for both combined drugs. The aqueous solubility of paracetamol could be significantly increased by using ethanol as second solvent.

Therewith, according to one embodiment, the formulation is in a solubilized and/or dissolved form in a solvent or a solvent mixture. In a preferred embodiment, the solvent is selected from ethanol, PEG 300, PEG 400, glycerol, glycerol formal, propylene glycol, water, and any combination thereof. Typically, the solvents are pharmaceutically acceptable, not-toxic and accommodating to body fluids, water soluble solvents or mixture of such solvents. The use of co-solvents that has been previously employed to enhance the solubility of poorly soluble drugs and has been proved to be well executed and beneficial.

The liquid parenterally deliverable formulation of the invention is therewith preferably a co-solvent system comprising mixtures of ethanol, PEG 300, PEG 400, propylene glycol and glycerol formal, mixed in satisfactory proportions that provides the integrity, stability and suitability of the liquid formulations for injectable use.

Accordingly, a PEG 400-ethanol mixture is particularly preferred as solvent. This system had high solubilization potential, which has significantly increased when in this solvent system glycerol formal has been added. Another preferred solvent system is ethanol-glycerol formal. A further preferred solvent system is ethanol-glycerol formal-propylene glycol. Another preferred solvent system is ethanol-glycerol formal-propylene glycol-polyethylene glycol 400. Yet another preferred solvent system is ethanol-glycerol formal-polyethylene glycol 300-glycol-polyethylene glycol 400.

A pH-solubility profile of paracetamol was obtained in the pH range 4 to 7. Therefore, in a particularly preferred embodiment, the formulation has a pH-value from 4 to 7, in particular 5 to 7, and more particular 6.5.

This can be obtained by using acceptable pharmaceutically buffers, in particular acceptable pharmaceutically buffer solutions. Therefore, in another embodiment, the liquid parenterally deliverable formulation additionally comprises a pharmaceutically acceptable buffer, in particular wherein the pharmaceutically acceptable buffer is selected from phosphate buffers, in particular disodium phosphate dodecahydrate; sodium carbonate; sodium bicarbonate; potassium carbonate; potassium bicarbonate; lithium carbonate; lithium bicarbonate; and any combination thereof.

The liquid formulations also includes stabilizing agents that further guarantees the indispensable for injectable administration terms and presuppositions, like antioxidants.

Therefore, in another embodiment, the liquid parenterally deliverable formulation additionally comprises an antioxidant, in particular wherein the antioxidant is selected from sodium metabisulfite, phosphorous acid, sodium sulfite, sodium bisulfite, sodium thiosulfate, and any combination thereof.

In yet another embodiment, the liquid parenterally deliverable formulation additionally comprises a chelating agent, in particular wherein the chelating agent is selected from ethylenediaminetetraacetic acid (EDTA), nitrilotriacetic acid, ethylenediamine-N,N'-diacetic-N,N'-dipropionic acid, and any combination thereof.

The liquid parenterally deliverable formulation can additionally comprise a local anesthetic, in particular Lidocaine hydrochloride.

In a particularly preferred embodiment the liquid parenterally deliverable formulation additionally comprises sodium metabisulfite, the chelating agent EDTA and selectively the local anaesthetic Lidocaine.

Typically, the formulation is provided in a sealed containe. In particular, the container is selected from a vial, an ampoule or a soft bag.

According to a further aspect, there is provided a kit comprising a liquid parenterally deliverable formulation as defined above and instructions for use of said formulation.

### Method of preparation

According to a another aspect, there is provided a method of producing a formulation a liquid parenterally deliverable formulation as defined above.

In particular, the method of producing a liquid parenterally deliverable formulation comprises
- a.: providing paracetamol, the tramadol material, optionally a pharmaceutically acceptable buffer as defined above, optionally an antioxidant as defined above, optionally a chelating agent as defined above, and optionally a local anesthetic as defined above; and
- b.: dissolving the materials provided in step a. in a solvent or solvent mixture as defined above.

A process for preparing the liquid parenteral formulation object of this invention comprises in a preferred embodiment the two steps :
A. Combining in specific order and mixing :
   (a) therapeutic dosage forms of paracetamol and tramadol HCl,
   (b) solubilizers effective to solubilize mainly paracetamol, said solubilizers being substantially ethanol, glycerol formal, PEG 400, propylene glycol,
   (c) a buffer solution determining the pH solubility profile of the active ingredients,
   (d) stabilizer components of the liquid parenteral formulation comprising an oxygen limiting agent and a chelating factor,
   (e) optionally Lidocaine HCl.
B. Dissolving paracetamol and tramadol HCl in a solvent mixture of the components of (b), (c), (d) and (e) of step A, whereby a required effective dosage is provided in sealed airtight container, which is selected from the group consisting of a vial or an ampoule.

The present invention is further illustrated with reference to the following examples, which are not intended to be in any way limiting to the scope of the invention. Accordingly, the scope of the invention should be determined not by the embodiments illustrated but by the appended claims and their legal equivants.

### EXAMPLE 1

| **PARACETAMOL-TRAMADOL LOT 09-01D** | | |
|---|---|---|
| **Ingredient** | **Quantity/1ml** | **Quantity /4 ml** |
| **Active ingredient** | | |
| paracetamol (mg) | 81,25 | 325 |
| tramadol HCl (mg) | 9,375 | 37,5 |

| **Excipients** | | |
|---|---|---|
| Glycerol formal (mg) | 595 | 2380 |
| Ethanol (mg) | 80 | 320 |
| Sodium Metabisulfite (mg) | 1 | 4 |
| Disodium Edetate (mg) | 0,1 | 0,4 |
| Disodium phosphate Dodecahydrate (mg) | 0,75 | 3 |
| Water for Injection to(ml) | QS | QS |
| Final pH (HCl or NaOH) 1M | 6,5 | |

### EXAMPLE 2

| **PARACETAMOL-TRAMADOL LOT 09-02D** | | |
|---|---|---|
| **Ingredient** | **Quanity/1ml** | **Quantity /4 ml** |
| **Active ingredient** | | |
| paracetamol (mg) | 81,25 | 325 |
| tramadol HCl (mg) | 9,375 | 37,5 |

| **Excipients** | | |
|---|---|---|
| Glycerol formal (mg) | 495 | 1980 |
| Propylene glycol (mg) | 100 | 400 |
| Ethanol (mg) | 80 | 320 |
| Sodium Metabisulfite (mg) | 1 | 4 |
| Disodium Edetate (mg) | 0,1 | 0,4 |
| Disodium phosphate Dodecahydrate (mg) | 0,75 | 3 |
| Water for Injection to(ml) | QS | QS |
| Final pH (HCl or NaOH) 1M | 6,5 | |

### EXAMPLE 3

| **PARACETAMOL-TRAMADOL LOT 09-03D** | | |
|---|---|---|
| **Ingredient** | **Quantity /1ml** | **Quantity /4 ml** |
| **Active ingredient** | | |
| paracetamol (mg) | 81,25 | 325 |
| tramadol HCl (mg) | 9,375 | 37,5 |

| **Excipients** | | |
|---|---|---|
| Glycerol formal (mg) | 495 | 1980 |
| Polyethylene glycol 300 (PEG 300) (mg) | 50 | 200 |
| Polyethylene glycol 400 (PEG 400) (mg) | 50 | 200 |
| Ethanol(mg) | 80 | 320 |
| Sodium Metabisulfite (mg) | 1 | 4 |
| Disodium Edetate (mg) | 0,1 | 0,4 |
| Disodium phosphate Dodecahydrate (mg) | 0,75 | 3 |
| Water for Injection to(ml) | QS | QS |
| Final pH (HCl or NaOH) 1M | 6,5 | |

### EXAMPLE 4

| **PARACETAMOL-TRAMADOL LOT 09-04D** | | |
|---|---|---|
| **Ingredient** | **Quantity /1ml** | **Quantity/4 ml** |
| **Active ingredient** | | |
| paracetamol (mg) | 81,25 | 325 |
| tramadol HCl (mg) | 9,375 | 37,5 |

| **Excipients** | | |
|---|---|---|
| Glycerol formal (mg) | 495 | 1980 |
| Propylene glycol (mg) | 50 | 200 |
| Polyethylene glycol 400 (PEG 400) (mg) | 50 | 200 |
| Ethanol (mg) | 80 | 320 |
| Sodium Metabisulfite (mg) | 1 | 4 |
| Disodium Edetate (mg) | 0,1 | 0,4 |
| Disodium phosphate Dodecahydrate (mg) | 0,75 | 3 |
| Water for Injection to(ml) | QS | QS |
| Final pH (HCl or NaOH) 1M | 6,5 | |

## Claims

1. A liquid parenterally deliverable formulation comprising a tramadol material and paracetamol.

2. The formulation of claim 1, wherein the tramadol material is a tramadol salt, in particular selected from tramadol hydrochlorid, tramadol hydroiodide, tramadol tartrate, tramadol citrate, tramadol methane sulphonate, and any combination thereof.

3. The formulation of claims 1 or 2, wherein the formulation comprises from 20 mg to 50 mg, particularly 30 mg to 40 mg, and more particularly 37,5 mg tramadol material.

4. The formulation of any of claims 1 to 3, wherein the formulation comprises from 200 mg to 500 mg, particularly from 300 mg to 400 mg, and more particularly 325 mg paracetamol.

5. The formulation of any of claims 1 to 4, wherein the formulation is in a solubilized and/or dissolved form in a solvent.

6. The formulation of claim 5, wherein the solvent is selected from ethanol, PEG 300, PEG 400, glycerol, glycerol formal, propylene glycol, water, and any combination thereof.

7. The formulation of any of claims 1 to 6, additionally comprising a pharmaceutically acceptable buffer, in particular wherein the pharmaceutically acceptable buffer is selected from phosphate buffers, in particular disodium phosphate dodecahydrate; sodium carbonate; sodium bicarbonate; potassium carbonate; potassium bicarbonate; lithium carbonate; lithium bicarbonate; and any combination thereof.

8. The formulation of any of claims 1 to 7, wherein the formulation has a pH-value from 4 to 7, in particular 5 to 7, and more particular 6.5.

9. The formulation of any of claims 1 to 8, additionally comprising an antioxidant, in particular wherein the antioxidant is selected from sodium metabisulfite, phosphorous acid, sodium sulfite , sodium bisulfite, sodium thiosulfate, and any combination thereof.

10. The formulation of any of claims 1 to 9, additionally comprising a chelating agent, in particular wherein the chelating agent is selected from ethylenediaminetetraacetic acid (EDTA), nitrilotriacetic acid, ethylenediamine-N,N'-diacetic-N,N'-dipropionic acid, and any combination thereof.

11. The formulation of any of claims 1 to 10, additionally comprising a local anesthetic, in particular Lidocaine hydrochloride.

12. The formulation of any of claims 1 to 11, wherein the formulation is provided in a sealed container, in particular wherein the container is selected from a vial, an ampoule or a soft bag.

13. A kit comprising a formulation of any of claims 1 to 12 and instructions for use of said formulation.

14. A method of producing a formulation of any of claims 1 to 12, comprising
a. providing paracetamol, a tramadol material, optionally a pharmaceutically acceptable buffer, optionally as defined in claim 7, optionally an antioxidant, optionally as defined in claim 9, optionally a chelating agent, optionally as defined in claim 10, and optionally a local anesthetic, optionally as defined in claim 11,
b. dissolving the materials provided in step a. in a solvent as defined in claims 5 or 6.
